(19) Europäisches Patentamt
European Patent Office
Office européen des brevets

(11) **EP 0 711 565 B1**

(12) **EUROPEAN PATENT SPECIFICATION**

(45) Date of publication and mention
of the grant of the patent:
**26.08.1998 Bulletin 1998/35**

(51) Int. Cl.⁶: **A61K 39/395**, A61K 48/00,
G01N 33/574

(21) Application number: **95117465.5**

(22) Date of filing: **07.11.1995**

(54) **Inhibiting growth of leukemic cells by targeting HER-2 protein**

Verfahren zur Wachstumshemmung von Leukämischen Zellen durch HER-2-Protein-Zeilen

Inhibition de la croissance des cellules leucemiques par ciblage de la protéine HER-2

(84) Designated Contracting States:
**AT BE CH DE ES FR GB IT LI NL SE**

(30) Priority: **10.11.1994 US 337605**

(43) Date of publication of application:
**15.05.1996 Bulletin 1996/20**

(73) Proprietor:
**Eberhard-Karls-Universität Tübingen
Universitätsklinikum
72076 Tübingen (DE)**

(72) Inventor:
**Bühring, Hans-Jörg, Dr.
D-72076 Tübingen (DE)**

(74) Representative:
**Otten, Hajo, Dr.-Ing. et al
Witte, Weller, Gahlert, Otten & Steil,
Patentanwälte,
Rotebühlstrasse 121
70178 Stuttgart (DE)**

(56) References cited:
- LEUKEMIA & LYMPHOMA, vol. 4, no. 5/6, 1991 pages 419-422, XP 000560889 NOBUTAKA IMAMURA ET AL. 'Aggressive diffuse lymphoma coexpressing NRAS p21 and C-erbB-2 (neu) oncogene products, and CALLA (CD10)'
- JOURNAL OF CLINICAL IMMUNOLOGY, vol. 11, no. 3, May 1991 pages 117-127, XP 000560916 H. MICHAEL SHEPARD ET AL. 'Monoclonal antibody therapy of human cancer: taking the HER2 protooncogene to the clinic'
- ACTA ONCOLOGICA, vol. 32, no. 7/8, 1993 pages 709-715, XP 000561701 STEVEN M. LARSON ET AL. 'Recent achievements in the development of radiolabeled monoclonal antibodies for diagnosis, therapy and biologic characterization of human tumors '
- BLOOD, vol. 86, no. 5, 1 September 1995 pages 1916-1923, XP 000560897 HANS JÖRG BÜHRING ET AL. 'The receptor Tyrosine Kinase p185HER2 is expressed on a subset of B-lymphoid blasts from patients with acute lymphoblastic leukemia and chronic myelogenous leukemia'
- Pschyrembel Kiinisches Wörterbuch, 257th edition, pages 869-870, 914-915
- Encyclopedia of Immunology, 1992, pages 1016-1017

**Description**

FIELD OF THE INVENTION

The present invention concerns methods for inhibiting the growth of leukemic cells and treating leukemia patients.

BACKGROUND OF THE INVENTION

Leukemia is a neoplastic condition of hematopoietic stem cells. Leukemic cells, or blasts, are generally classified as having either lymphoid or myeloid differentiation. However, blasts may also be undifferentiated or have both lymphoid and myeloid features. Leukemia is also classified as being acute or chronic.

Hematopoietic stem cells differentiate to give rise to "terminally" differentiated cells such as neutrophils, erythrocytes, platelets, and plasma cells. (See Bagby, G. C., Jr., Chapter 3, *The molecular Basis of Blood disease* 2$^{ed}$, W.B. Saunder Company, 1994.) Acute leukemia results from the malignant transformation of a hematopoietic stem cell producing a clone of cells characterized by disordered growth and maturation. The clone has a selective growth advantage over normal cells and eventually supplants normal cells in the bone marrow. Differentiation is limited in the clone cells and cells not maturing may be morphologically and functionally abnormal. Thus, maturation may be "frozen" at an early stage of development with normal hematopoiesis replaced by a mechanism making a product that is both quantitatively and qualitatively altered.

Acute leukemia results in bone marrow failure and organ infiltration by malignant leukemic cells. (Reviewed by O'Rourke and Kalter *Leukemia*, In: *Clinical Oncology*, Chapter 28, Eds. Weiss, Appleton and Lange; Norwalk Conn, 1993.) Thus, in addition to adversely affecting hematopoiesis, blasts can infiltrate other organs such as the central nervous system, skin, liver, spleen, or gastrointestinal tract, leading to local symptoms or organ failure. Chronic leukemia differs from acute leukemia by its onset and prolonged course. Often chronic leukemia is present with few or no symptoms (Reviewed by O'Rourke and Halter, Supra.)

In the publication "Agressive diffuse lymphoma coexpressing NRASp21 and C-ErbB-2 (neu) oncogene products, and CALLA (CD10)" by Imamura, N. et al., in Leukemia and Lymphoma, Vol. 4, pp. 419-422, the authors demonstrate the overexpression of HER-2 (also C-ErbB-2 or neu) on malignant lymphoma cells derived from one patient suffering from agressive diffuse lymphoma. However, no hint regarding the overexpression of HER-2 on leukemic cells is given in this document.

SUMMARY OF THE INVENTION

The present invention identifies a subset of leukemic cells which express HER-2 protein on their cell surface. The invention features methods for inhibiting the growth of such cells and treating leukemia patients by targeting the HER-2 protein. The HER-2 protein is targeted using different techniques such as using anti-HER-2 agents which inhibit HER-2 protein activity and the growth of cells expressing HER-2, using anti-HER-2 agents which bind to HER-2 protein and kill cells expressing HER-2 protein, and using HER-2 protein binding agents to selectively remove leukemic cells expressing HER-2 from normal hematopoietic cells.

HER-2 protein expression was found on the surface of leukemic cells obtained from patients characterized as having common acute lymphoblastic leukemia (C-ALL) or chronic myelogenous leukemia (CML) in lymphoid blast crises, and in leukemic cell lines. All of the leukemic cells expressing HER-2 protein also expressed CD19 which is a surface antigen characteristic of the B-cell lineage. Expression of HER-2 protein in a leukemic cell indicates that the enhanced or uncontrolled proliferation characteristic of the leukemic cell is to some extent driven by HER-2 protein activity.

HER-2 protein, also know as p185(HER-2), is a receptor tyrosine kinase protein. "HER-2 protein expression" refers to an increased amount of HER-2 protein on the cell surface. The increased amount is relative to that occurring in non-leukemic hematopoietic cells. As described in the examples below, none of the non-leukemic hematopoietic cells tested had detectable expression of HER-2 protein on their cell surface.

Anti-HER-2 agents are made up of molecules or compounds targeted to cells expressing HER-2 protein which either inhibits the growth of, or kills, cells expressing HER-2 protein. Compounds targeted to cells expressing HER-2 protein exert a greater growth inhibitory effect or killing effect on a cell expressing HER-2 protein than a cell not expressing HER-2 protein. Targeting cells expressing HER-2 protein can be achieved using different techniques such as using agents able to bind to HER-2 protein and/or able to inhibit one or more HER-2 protein activities. HER-2 protein activities are cellular processes mediated or effected by HER-2 protein. Examples of anti-HER-2 agents include HER-2 inhibiting compounds, anti-HER-2 antibodies, anti-HER-2 nucleic acid, and defective HER-2 protein.

Thus, a first aspect of the present invention features a method for inhibiting proliferation of a leukemic cell expressing HER-2 protein. The method can be carried out *in vivo* or *in vitro* and involves exposing the leukemic cell to a proliferation inhibiting amount of an anti-HER-2 agent.

A "proliferation inhibiting amount" of an anti-HER-2 agent is an amount of an agent which inhibits the growth of, or kills, a leukemic cell expressing HER-2 protein to greater extent than it inhibits the growth of, or kills, a non-leukemic hemopoietic cell. Preferably, it is an amount which completely inhibits the growth of, or kills, a leukemic cell expressing HER-2 protein while having little or no effect on non-leukemic hemopoietic cells.

In a preferred embodiment, the method is used to treat a patient suffering from a HER-2 leukemia of the B-lymphoblastic lineage. A "HER-2 leukemia" refers to the presence of leukemic cells expressing HER-2 protein. "B-lymphoblastic linage" refers to B-cells of various maturational stages.

The development of stem cells to mature B cells can be characterized by the acquisition and loss of B-lineage polypeptides. In a preferred embodiment the leukemic cell expresses CD10 or CD19. By "expresses" in reference to a polypeptide is meant that the presence of the polypeptide is detectable on the cell surface using standard techniques such as immunofluorescence staining.

Another aspect of the present invention features an *ex vivo* method of treating a leukemia patient having a leukemic cell expressing HER-2 protein. The method involves the steps of: (a) removing hematopoietic cells containing leukemic cells expressing HER-2 protein from a patient; (b) exposing the cells to a proliferation inhibiting amount of an anti-HER-2 agent, and (c) returning the treated hematopoietic cells to the patient. Preferably, the hematopoietic cells are expanded before being returned to the patient.

Hematopoietic cells can be removed from, and returned to, a patient using standard techniques. Peters, *Transplantation*, In: *Clinical Oncology*, Chapter 13, Eds. Weiss, Appleton and Lange, Norwalk Conn, 1993 (hereby incorporated by reference herein). Preferably, the removed cells are enriched for leukemic cells of B-lymphoblastic lineage prior to undergoing treatment. Enrichment can be carried out using an antibody affinity column which bind to cell surface markers characteristic of B-lymphoblastic lineage.

Another aspect of the present invention features a method for treating a leukemia patient having a leukemic cell expressing HER-2 protein by filtering out the leukemic cells. Filtering results in the selective removal of leukemic cells expressing HER-2 protein from a solution containing leukemic cells and normal hematopoietic cells. As a result of such filtration, the ratio of leukemic cells expressing HER-2 protein to normal hematopoietic cells is decreased and preferably all leukemic cells expressing HER-2 are eliminated.

Selective removal can be carried out using agents which bind to cell surface HER-2 protein but do not appreciable bind cells not expressing HER-2 protein. Such agents include anti-HER-2 compounds binding HER-2 protein, natural HER-2 protein ligands, and antibodies binding to HER-2 protein. Preferably, the method is carried out by passing hematopoietic cells comprising leukemic cells through an antibody affinity column containing antibodies binding HER-2 protein.

Another aspect of the present invention features an *in vivo* method of treating a patient having a leukemic cell expressing HER-2 protein. The method involves administering a therapeutically effective amount of an anti-HER-2 agent to the patient.

A "therapeutically effective amount" refers to an amount which confers an overall beneficial effect to a patient by achieving one or more or the following: 1) reduction in the number of leukemic cells; 2) inhibition (*i.e.*, slowing to some extent, preferably stopping) of cancer cell infiltration into peripheral organs; 3) inhibition of leukemic cell growth, preferably killing of leukemic cells; and 4) relieving to some extent one or more symptoms associated with the leukemia.

Preferably, a therapeutic anti-HER-2 agent is administered to a patient with a physiologically acceptable carrier. A physiologically acceptable carrier is a formulation to which the agent can be added to dissolve it or otherwise facilitate its administration. Examples of physiologically acceptable carriers include water, saline, physiologically buffered saline and cyclodextrins. An important factor in choosing an appropriate physiologically acceptable carrier is choosing a carrier in which the agent remains active or the combination of the carrier and the agent produces an active agent.

Another aspect of the present invention features a method for determining whether a patient has a leukemia characterized by the cell surface expression of HER-2 protein. The method involves the step of providing a HER-2 binding agent to hemopoietic cells containing leukemic cells and measuring the binding of the agent to the leukemic cells. For example, bone marrow cells can be removed from a leukemia patient, contacted with an anti-HER-2 antibody, and binding of the antibody to a leukemic cell expressing HER-2 protein can be measured.

Another aspect of the present invention features a method for determining whether a patient has a leukemia which is driven by HER-2 protein activity. The method involves the Steps of: (a) obtaining leukemic cells from a patient; (b) exposing the cells to a cell proliferation inhibiting amount of an anti-HER-2 agent, and (c) measuring the growth of treated cells.

Thus, the present invention allows for the treatment of leukemia by identifying a therapeutic target site: HER-2 protein. Prior to identifying the presence of HER-2 protein expression in a leukemic cell it was not known that the growth of such cells could be inhibited by targeting HER-2 protein.

Other features and advantages of the invention will be apparent from the following description of the preferred embodiments thereof, and from the claims.

DETAILED DESCRIPTION OF THE INVENTION

The present invention links HER-2 protein expression to certain classes of leukemic cells and, thereby, allows for targeting of HER-2 protein to inhibit the growth of such cells and to treat leukemia patients. HER-2 protein surface expression and HER-2 mRNA expression were examined in different cells including leukemic cells obtained from leukemia patients and hematopoietic cells obtained from healthy patients.

HER-2 protein expression was detected in some leukemic blasts of the B-lymphoblastic lineage, but was absent in normal cells of hematopoietic origin. HER-2 mRNA expression was found in normal peripheral blood mononuclear cells, bone marrow cells, and all of the leukemic cells and cell lines tested. However, cells expressing HER-2 protein showed a pronounced increase in HER-2 mRNA compared to normal cells as measured by semi-quantitative PCR analysis. These results demonstrate that HER-2 protein is a leukemic-specific cell surface marker which is not found on blood cells from healthy donors.

## I. LEUKEMIC CELLS EXPRESSING HER-2 PROTEIN

Different types of leukemic cells expressing HER-2 protein can be identified and treated by targeting HER-2 protein. Leukemic cells expressing HER-2 protein can be identified using standard techniques such as by exposing the cells to an antibody recognizing HER-2 protein. HER-2 leukemic cells identified, thus far, are of the B-lymphoblastic lineage as determined by the presence of CD19 and in most cases also CD10. B-lymphoblastic lineage cells can be identified by characteristic antigens and using standard classification schemes. See, Mitus and Rosenthal, *Adult Leukemia*; In: *American Society Textbook of Clinical Oncology*, chapter 30, Eds. Holleb, Fink, and Murphy, American society Inc. 1991; and Pui and Rivera, *Infant Leukemia;* In: *American Society Textbook of Clinical Oncology*, chapter 31, Eds. Holleb, Fink, and Murphy, American Society Inc. 1991;.

### A. HER-2 Protein Activity

The HER-2 protein is a member of the class I receptor tyrosine kinase (RTK) family. Yarden and Ullrich, *Annu. Rev. Biochem. 57*:443, 1988; Ullrich and Schlessinger, *Cell 61*:203, 1990. HER-2 protein is structurally related to EGF-R, p180(HER-3), and p180(HER-4). Carraway, *et al., Cell 78*:5, 1994; Carraway, *et al., J. Biol. Chem. 269*:14303, 1994. These receptors share a common molecular architecture and contain two cysteine-rich regions within their cytoplasmic domains and structurally related enzymatic regions within their cytoplasmic domains.

Activation of HER-2 protein can be caused by different events such as ligand-stimulated homo-dimerization, ligand-stimulated hetero-dimerization and ligand-independent homo-dimerization. Ligand-stimulated hetero-dimerization appears to be induced by EGF-R to form EGF-R/HER-2 complexes and by neu differentiation factor/heregulin (NDF/HRG) to form HER-2/HER-3 and/or HER-2/HER-4 complexes. Wada *et al., Cell 61*:1339, 1990; Slikowski *et al., J. Biol. Chem. 269*:14661, 1994; Plowman *et al., Nature 266*:473, 1993. Ligand-dependent activation of HER-2 protein is thought to be mediated by neu-activating factor (NAF) which can directly bind to p185(HER-2) and stimulate enzymatic activity. Dougall *et al., Oncogene 9*:2109, 1994; Samata *et al., Proc. Natl. Acad. Sci. USA 91*:1711, 1994. Ligand-independent homo-dimerization of HER-2 protein and resulting receptor activation is facilitated by over-expression of HER-2 protein.

Receptor tyrosine kinases are involved in various cellular signaling pathways. Receptor phosphorylation stimulates a physical association of the activated receptor with target molecules. Receptor tyrosine kinase substrates and adaptor proteins (also know as docking proteins) are both involved in signal transduction from an activated receptor tyrosine kinase. A receptor tyrosine kinase substrate is phosphorylated by the receptor tyrosine kinase and can then act on another cellular protein. An adaptor protein, such as Grb-2, binds a receptor tyrosine kinase and to another protein helping to activate the other protein. Substrates and adaptor proteins typically bind to receptor tyrosine kinases by SH2 and SH3 domains. Pawson and Schlessinger, *Current Biology 3*:434, 1993.

HER-2 protein substrates are acted upon by activated HER-2 complexes such as HER-2/EGF-R, HER-2/HER-2, HER-2/HER-3, and HER-2/HER-4 activated complexes. An activated HER-2 complex acts as a phosphokinase and phosphorylates different cytoplasmic proteins. Examples of HER-2 substrates include, $IP_3$ kinase and PI 4-kinase. Scott *et al., Journal of Biological Chemistry 22*:14300, 1991.

HER-2 adaptor proteins bind to an activated HER-2 complex and then another protein. For example, GRB-7 binding to a HER-2 complex may be sufficient to initiate the GRB-7 signaling pathway without phosphorylation. Stein *et al., EMBO Journal 13*:1331, 1993.

Thus, HER-2 protein activities include: (1) phosphorylation of HER-2 protein, HER-3 protein or HER-4 protein; (2) phosphorylation of a HER-2 protein substrate; (3) interaction with a HER-2 adapter protein; and/or (4) HER-2 protein surface expression. Anti-HER-2 agents preferably bind HER-2 protein and inhibits one or more HER-2 protein activities and/or causes leukemic cell death.

Additional HER-2 protein activities can be identified using standard techniques. For example, a partial agonistic monoclonal antibody recognizing HER-2 protein can be used to activate HER-2 protein and examine signal transduction of HER-2 protein. Scott *et al., Journal of Biological Chemistry 22*:14300, 1991.

B. Over-Expression of the Gene Encoding HER-2 protein

Over-expression of the gene encoding HER-2 protein has been linked to neoplastic transformations in different cells by different investigators. Slamon *et al., Science 244*:707, 1989, examined the correlation between HER-2/*neu* and breast and ovarian carcinoma, and also examined procedures used to measure the correlation. According to Slamon:

The HER-2/*neu* proto-oncogene is amplified in 25 to 30 percent of human primary breast cancers and this alteration is associated with disease behavior. In this report, several similarities were found in the biology of HER-2/*neu* in breast and ovarian cancer, including a similar incidence of amplification, a direct correlation between amplification and over-expression, evidence of tumors in which overexpression occurs without amplification, and the association between gene alteration and clinical outcome. A comprehensive study of the gene and its products (RNA and protein) was simultaneously preformed on a large number of both tumor types. This analysis identified several potential shortcomings of the various methods used to evaluate HER-2/*neu* in these diseases (Southern, Northern, and Western blots, and immunohistochemistry) and provided information regarding considerations that should be addressed when studying a gene or gene product in human tissue. The data presented further support the concept that the HER-2/*neu* gene may be involved in the pathogenesis of some human cancers.

Scott *et al., Journal of Biological Chemistry 22*:14300, 1991, examined p185(HER-2) signal transduction in breast cancer cells. In discussing prior work done in the field Scott *et al* asserts:

Amplification and/or overexpression of HER-2/*neu* has been detected in gastrointestinal, nonsmall cell lung, and ovarian adenocarcinomas and occurs in a significant fraction of primary human breast cancers where it correlates with regionally advanced disease, increased probability of tumor recurrence, and reduced patient survival. (Citations omitted).

Shepard *et al., J. clinical Immunology 11*:117, 1991, also refers to clinical and experimental evidence supporting a role for overexpression of HER-2 protooncogene in the progression of human breast, ovarian, and non-small cell lung carcinoma.

II. ANTI-HER-2 AGENTS

Targeting of anti-HER-2 agents to cells expressing HER-2 protein can be achieved by targeting HER-2 protein itself or one or more HER-2 protein activities. Anti-HER-2 agents are selective for HER-2 expressing cells in that they either kill or inhibit cellular growth of HER-2 expressing cells to a greater extent than they affect equivalent cells not expressing HER-2 protein. Equivalent cells are non-leukemic hematopoietic cells. Preferably, the effect on HER-2 expressing cell is at least 10 fold greater than on equivalent cells, more preferably at least 100 fold greater, more preferably at least 1,000 fold greater, at one or more concentration of an anti-HER-2 agent. The magnitude of an effect can be measured using standard techniques such as by measuring the dose required to achieve 50% growth inhibition or killing.

Anti-HER-2 agents include HER-2 inhibiting compounds, anti-HER-2 antibodies, anti-HER-2 nucleic acid, and defective HER-2 protein. Inhibition of HER-2 protein activities can be assayed by measuring the effect of a HER-2 protein agonist on cellular processes in the presence and absence of an anti-HER-2 agent. Inhibiting growth of leukemic cells expressing HER-2 protein can be assayed using standard techniques such as by measuring the growth of the cells in the presence and absence of an anti-HER-2 agent. The amount of killing can be assayed using standard techniques to measure cell death such as using an MTT assay as described by Mossman *J. Immunol. Methods 65*:55-63 (1983), and by measuring the amount of LDH released (Korzeniewski and Callewaert, *J. Immunol. Methods 64*:313 (1983); Decker and Lohmann-Matthes, *J. Immunol. Methods 115*:61 (1988).

A. Anti-HER-2 Nucleic Acid

Anti-HER-2 nucleic acid inhibits HER-2 protein activity by either inhibiting production of protein involved in one or more HER-2 protein activities or through the production of a defective HER-2 protein. Examples of protein involved in HER-2 activity include the HER-2 protein itself, HER-2 substrates, and HER-2 adaptor proteins.

Different types of nucleic acids can be designed to inhibit HER-2 protein production. For example, anti-HER-2 oligonucleotides can be designed to act through an anti-sense mechanism or as a ribozyme to prevent protein production from nucleic acid encoding HER-2. Such oligonucleotides can be designed to contain a nucleic acid region perfectly complementary to a nucleic acid sequence encoding HER-2 protein to allow for targeting of HER-2 protein activity.

Antisense oligonucleotides can hybridize to a target RNA, such as mRNA, and inhibit protein production from that RNA. Anti-sense oligonucleotides may function by a variety of mechanisms. Proposed mechanisms include forming a DNA:RNA substrate for cellular RNase H, hybridization of an antisense oligonucleotide to nascent mRNA leading to premature transcription termination and interfering with mRNA processing by hybridizing to a pre-mRNA intron/exon junction. Helene, C. and Toulme, J. *Biochimica et Biophysica Acta 1049*:99 (1990), and Uhlmann, E. and Peyman, A. *Chemical Reviews 90*:543 (1990).

B. Anti-HER-2 Compounds

Compounds able to inhibit HER-2 protein activity are known in the art. For example, Gazit *et al., J. Med. Chem. 34*:1896-1907 (1991) and Gazit *et al., J. Med. Chem. 36*:3556-3564 (1993), describe compounds able to inhibit HER-2 protein activity. In the later article, Gazit *et al* describes tyrphostins having a *S*-aryl substituent in the 5 position and asserts:

We find that these compounds are potent blockers of EGFR kinase and its homolog HER-2 kinase. Interestingly, we find that certain *S*-aryltyrphostins discriminate between EGFR and HER--2 kinase in favor of the HER-2 kinase domain by almost 2 orders of magnitude. When examined in intact cells it was found that these selective *S*-aryltyrphostins are equipotent in inhibiting EGF dependent proliferation of NIH 3T3 harboring either the EGF receptor or the chimera EGF/neu HER-1-2.

Osherov *et al., Journal of Biological Chemistry 268*:11134, 1993, mentions the development of two groups of tyrphostins:

one is highly selective in inhibiting HER-1 [EGF] as compared with HER-2 kinase activity, and the other is highly selective in inhibiting HER-2 activity compared with HER-1 kinase activity.

Other anti-HER-2 compounds can be obtained by measuring the ability of compounds to inhibit HER-2 protein activity and the growth of leukemic cells expressing HER-2 protein.

C. Anti-HER-2 antibodies

Antibodies able to bind HER-2 protein can be used to inhibit the growth of cells expressing HER-2 protein in different ways including the modulation of HER-2 activity and targeting of toxic moieties to a cell expressing HER-2 protein. For example, monoclonal antibodies with specificity for HER-2 protein having semi-agonistic activities can be used to inhibit the growth of leukemic cells expressing HER-2 protein. The functional properties of semi-agonistic antibodies differ from that of natural ligands by their ability to cause incomplete activation of the cellular signalling cascade.

Antibodies able to act as anti-HER-2 agents which may work by modulating HER-2 activity are described by Hudziak *et al., Mol. Cell. Biol. 9*:1165, 1989; Sarup *et al., Growth Regulation 1*:71, 1991; and Shepard *et al., J. clinical Immunology 11*:117, 1991. Shepard *et al.*, describes the production of antibodies which recognize HER-2 protein and not EGF-R. Such antibodies are preferred for therapeutic *in vivo* uses because EGF-R is closely related to HER-2 protein and is expressed at elevated levels in multiple tissues.

Antibodies can also be used as binding agents targeted to HER-2 to facilitate cell death. Examples of toxins which can be conjugated to antibodies include gelonin, ricin, and shigella toxin, diphtheria toxin and *Pseudomonas* exotoxin A. Toxins can be conjugated to antibodies using standard techniques. Olsnes *et al., Immunology Today 10*:291, 1989.

D. Defective HER-2 protein

Defective HER-2 proteins are signaling incompetent derivatives of a HER-2 protein. Formation of a HER-2 complex involving a signaling incompetent HER-2 protein leads to the formation of a defective complex having reduced HER-2 protein activities.

Defective HER-2 protein contain one or more amino acid mutation resulting in a mutated HER-2 protein which can inhibit one or more activities of a HER-2 complex. Amino acid mutations include additions, deletions and substitutions. Examples of mutations resulting in defective HER-2 protein include those which inhibit receptor phosphorylation or binding to a substrate or an adaptor molecule. Defective HER-2 protein can inhibit receptor activity in different ways such as by participating in receptor dimerization with a functioning HER-2 protein and inhibiting receptor phosphorylation.

III. Therapeutic use

Anti-HER-2 agents can be used therapeutically to treat a patient having leukemic cells expressing HER-2 protein. A "patient" refers to a human having leukemic cells expressing HER-2 protein. Patients can be diagnosed as having leukemia using standard medical techniques. Leukemic cells from such patients can be tested to determine whether the leukemic cells express HER-2 protein. Patients having leukemic cells expressing HER-2 protein can then be treated *in vivo* or *ex vivo*. Factors influencing the use of different therapeutic compounds including proper dosage, dosage form, toxicity and the impact of patient parameters are known in the art. Goodman and Gillman, *The Pharmacological Basis of Therapeutics*, McMillan Publishing Co., Inc. New York 1980; *Remington's Pharmaceutical Science* 15th ed., Mack Publishing Company, Easton, Penn., 1980.

A. Combination Treatment

Anti-HER-2 agents can be used alone, in combination with other anti-HER-2 agents of the same or different type, and in combination with other types of leukemia treatments. Various different types of general treatments are currently used to treat different types of leukemia patients. These general treatments are based on the leukemia type and do not specifically target HER-2 protein.

For example, different chemotherapeutic agents are used to treat different types of leukemia. O'Rourke and Kalter *Leukemia*, In: *Clinical Oncology*, Eds. Weiss, Appleton and Lange; Norwalk Conn, 1993; Mitus and Rosenthal, *Adult Leukemia*, In: *American Society Textbook of Clinical Oncology*, chapter 30, Eds. Holleb, Fink, and Murphy; and Pui and Rivera, *Infant Leukemia*, In: *American Society Textbook of Clinical Oncology*, chapter 31, Eds. Holleb, Fink, and Murphy;. Examples of chemotherapeutic agents include treatment of AML using daunorubicin, cytarabine (Ara-C), doxorubicin, amsacrine, mitoxantrone, etoposide (VP-16), thioguanine, mercaptopurine, and azacytidine; treatment of ALL using vincristine, prednisone, doxorubicin and asparginase; treatment of CML using busulfan and hydroxyurea; and treatment of CLL using chlorambucil and cyclophosphamide. Additional treatments include use of alpha-interferon, bone marrow transplantation and transplantation of peripheral blood or umbilical cord blood stem cells.

An example of a combination treatment involving transplantation is autologous bone marrow transplantation used in conjunction with anti-HER-2 agents. The treatment could be carried out using the following steps: 1) peripheral blood cells or bone marrow cells are removed from a patient; 2) the removed cells are treated with a proliferation inhibiting amount of an anti-HER-2 agent; 3) the patient is irradiated to kill leukemic cells not removed; and 4) the treated cells are returned to the patient. The treated cells can be expanded prior to being returned to the patient.

Autologous bone marrow transplant can also be used in conjunction with anti-HER-2 binding agents. For example, an antibody which binds HER-2 can be used to filter out leukemic cells expressing HER-2 from hematopoietic cells. The filtered hematopoietic cells are returned to an irradiated patient.

B. *In Vivo* Treatment of Leukemia

Patients can be treated *in vivo* using a therapeutically effective amount of an anti-HER-2 agent. The chosen anti-HER-2 agent should not cause side effects which would be regarded as not tolerable to a patient. Proper administration of an anti-HER-2 agent brings the agent into contact with leukemic cells expressing HER-2 protein in a sufficient amount to exert a therapeutic effect.

The chosen method of administration should take into account factors affecting the proper functioning of the agent such as the type of anti-HER-2 agent, the type of patient, and the disease condition. Agents exerting their effect inside a cell such as anti-HER-2 nucleic acid need to be transported into the cell.

Anti-HER-2 agents can be administered by different routes including intravenous, intraperitoneal, intranasal, intrathecal, ophthalmic, subcutaneous, intramuscular, oral, topical, and transmuccosal. Standard drug delivery vehicles are available for different types of administration. There may be advantages to a particular route of administration for a particular agent, patient and disease.

Delivery routes such as intravenous, intraperitoneal, intranasal, intrathecal, ophthalmic, subcutaneous, intramuscular, oral, and transmuccosal can be chosen to allow for systemic adsorption. Systemic adsorption results in agent accumulation in the blood stream followed by distribution throughout the entire body.

Anti-HER-2 agents can be administered to a patient by itself, or in a pharmaceutical composition comprising the active compound and a carrier or excipient. Carriers or excipients can be used to facilitate administration of anti-HER-2 agents. For example, carriers or excipients such as calcium carbonate, calcium phosphate, various sugars or types of starch, cellulose derivatives, gelatin, vegetable oils, polyethylene glycols and physiologically compatible solvents can be use to increase the solubility of an anti-HER-2 compound.

Anti-HER-2 nucleic acid can be introduced into a cell using standard techniques such as those involving a retroviral vector, ion paired molecules, covalently attached adducts and liposomes. Liposomes are hollow spherical vesicles composed of lipids arranged in a similar fashion as those lipids making up the cell membrane. They have an internal aqueous space for entrapping water soluble compounds and range in size from 0.05 to several microns in diameter. Liposomes offer several advantages including increased intracellular stability and increased uptake efficiency. Additionally, antibodies can be attached to liposomes to target particular cells, such as cells expressing HER-2 protein.

Drug delivery vehicles can be designed to serve as a slow release reservoir, or to deliver their contents directly to the target cell. An advantage of using direct delivery drug vehicles is that multiple molecules are delivered per uptake.

Such vehicles increase the circulation half-life of drugs which would otherwise be rapidly cleared from' the blood stream. Examples of such specialized drug delivery vehicles are liposomes, hydrogels, cyclodextrins, biodegradable nanocapsules, and bioadhesive microspheres.

Different treatment regimes may be used to administer anti-HER-2 agents, such as frequent small daily doses or a few large daily doses. Small daily doses can be administered, for example, at short time intervals using a pump to control the time interval or achieve continuously administration. Suitable pumps are commercially available (*e.g.*, the ALZET® pump sold by Alza corporation, and the BARD ambulatory PCA pump sold by Bard MedSystems).

The proper dosage depends on various factors such as the type of disease being treated, the particular composition being used, and the size and physiological condition of the patient. The daily dose of an anti-HER-2 compound is expected to be between 1 to 2,000 mg/day, preferably 1 to 250 mg/day, and most preferably 10 to 150 mg/day. Compounds can be delivered less frequently provided plasma levels of the active moiety are sufficient to maintain therapeutic effectiveness.

Assays can be carried out to measure the ability of different doses of an agent to reach a target location and also to measure the ability of the agent to enter a cell. Efficacy and cytotoxicity can then be tested. Toxicity not only includes cell viability but also cell function. Uptake studies include uptake assays to evaluate cellular nucleic acid and protein uptake. Uptake studies can also be used to determine the intracellular localization of the agent following uptake, ultimately establishing the requirements for maintenance of steady-state concentrations within the cellular compartment containing the target sequence (nucleus and/or cytoplasm). Assays are preferably first carried out using animal models prior to use in humans.

### C. *Ex vivo* Treatment of Leukemia

Preferred *ex vivo* treatments involve removal of cells or tissues containing leukemic blasts from a patient, treating the removed cells or tissues using an anti-HER-2 agent and/or a HER-2 binding agent, and returning the treated cells or tissues back to the patient. Hemopoietic cells can be removed from a patient using standard techniques such as leukapheresis and withdrawal of bone marrow cells. Peters, *Transplantation*, In: *Clinical Oncology*, Chapter 13, Eds. Weiss, Appleton and Lange, Norwalk Conn, 1993. Anti-HER-2 agents are used to selectively inhibit proliferation of the leukemic cells. HER-2 binding agent are used to filter out HER-2 protein expressing cells.

When anti-HER-2 agents are used, hemopoietic cells of the B-lymphoblastic linage are preferably purified to some extent prior to using the anti-HER-2 agent. For example, antibodies to CD19 can be used to achieve a measure of purification.

Their are benefits and drawbacks to *ex vivo* treatment compared to *in vivo* treatment. For example, an advantage of *ex vivo* treatment is that HER-2 leukemia is directly exposed to the anti-HER-2 agent. As a result of direct exposure, considerations such metabolism of the agent in the body, transport of the agent through the body, and possible adverse side effects to healthy organs are diminished. Some disadvantages of *ex vivo* treatment result from it being a more invasive procedure requiring removal of cells from a patient, return of the cells to a patient, and only removed cells will be directly exposed to the agent.

The use of *ex vivo* treatment expands the techniques available to introduce anti-HER-2 agents into a cell. For example, techniques such as electroporation can be used to introduce nucleic acid into cells removed from a body.

### D. Filtering of Leukemic Cells Expressing HER-2 Protein

The present invention also provides for treating patients having leukemic cells expressing HER-2 protein by filtering out the leukemia cells expressing HER-2 protein using HER-2 binding agents. HER-2 binding agents bind to HER-2 protein to a greater extent than they bind to other cell surface markers. Preferably, the binding agents bind cells expressing HER-2 at least 100 fold greater, more preferably at least 1,000 greater, than they bind cells not expressing HER-2 protein. Most preferably, the binding agent binds cells expressing HER-2 protein and does not appreciable bind cells not expressing HER-2 protein.

Preferred binding agents are immunoglobulins targeted to HER-2 protein. The immunoglobulin may be a monoclonal or polyclonal antibodies and may comprise whole antibodies or immunologically reactive fragments of these antibodies such as $F_{(ab')}$, $F_{(ab)}$, or $(F_{ab'})_2$. Techniques to produce antibodies targeting HER-2 protein are known in the art. Scott *et al., Journal of Biological Chemistry 22*:14300, 1991; Shepard *et al., J. clinical Immunology 11*:117, 1991; *see also* Example 1, *infra*.

Less preferred *ex vivo* treatments target B-cell markers present in conjunction with HER-2 protein. These less preferred treatments target B-cell markers such as CD19. A drawback to this type of treatment is that it is not specific for just HER-2 protein expressing cells.

Filtration of leukemic cells expressing HER-2 protein can be carried out using standard techniques, such as using an antibody affinity column, using immunoadsorption, and using antibodies conjugated to magnetic beads. Filtration treatment is preferably carried out by removing hematopoietic cells comprising leukemic cells expressing HER-2 protein from a patient, passing the cells through an antibody affinity column which binds HER-2 protein, and reintroducing the filtered hematopoietic cells into the patient. If needed, removed hematopoietic cells can undergo numerous rounds of filtration.

The filtration method can be used as part of a treatment regime involving anti-HER-2 agents or general chemotherapeutic techniques. For example, general chemotherapeutic agents can be administered to patients who have undergone filtration treatment and filtered cells can be returned to patients who have undergone radiation treatment.

## EXAMPLES

Examples are provided below to illustrate different aspects and embodiments of the present invention. These examples are not intended in any way to limit the disclosed invention.

### Example 1: HER-2 Protein Expression In Patients

Normal hematopoietic cells and leukemic cells were tested for HER-2 protein expression using antibodies targeted to HER-2 protein. Normal hematopoietic cells and leukemic cells were obtained from infant ($\leq$ 18) and adult (> 18) patients. Some leukemic cells expressed HER-2 protein, while none of the normal hematopoietic cells expressed HER-2 protein. None of the leukemia samples expressed EGF-R as determined using standard techniques involving an antibody binding to EGF-R. The procedures used to measure HER-2 protein expression (Materials and Methods) and results of these experiments are described below.

### Materials and Methods

*Antibodies*

Antibodies to HER-2 protein, and cell surface antigens were produced using standard techniques or obtained from commercial sources. The following monoclonal antibodies (mAb's) were obtained from commercial sources: CALLA (CD10), Leu-12 (CD19) and HPCA-2-FITC (CD34) from Becton Dickinson, Heidelberg, Germany; IOM41-FITC (CD41) and glycophorin A-FITC from Immunotech, Marseille, France.

Several monoclonal antibodies with specificity for the extracellular domain of HER-2 protein were raised by immunizing Balb/c mice by subsequent injections of NIH/3T3 cells transfected with the human protooncogene HER-2. Fendley *et al., Cancer Research 50*:1550, 1990. Immunization and fusion protocols were carried out as described by Bühring *et al., Hybridoma 10*:77, 1991.

Monoclonal antibodies specifically recognizing the transfectant cell line but not NIH-3T3 cells transfected only with the expression vector were selected. Specificity was confirmed by indirect immunofluorescence analysis (FACS) and immunoprecipitation of HER-2 protein. Monoclonal antibody 24D2 (IgG1) was selected for screening by flow cytometric analysis because staining of cells with reagent resulted in optimal signals. Monoclonal antibodies 13A1 and 13D1 were selected for down-regulation experiments because they showed growth inhibitory activity on SKBr3 breast carcinoma cells.

*Cells Obtained from Patients*

Normal peripheral blood (PB) cells, bone marrow (BM) cells, and leukemia samples were drawn from human patients. Erythrocytes were analyzed by using 5 $\mu$l of unfractionated heparinized PB cells for each assay. Platelets were obtained by collecting the plasma after settling of erythrocytes for 30 minutes and centrifugation at 2,000 rpm. The purity of erythrocyte and platelet populations were confirmed by labeling erythrocytes with the erythrocyte-specific marker glycophorin A and the platelets with the platelet-specific marker CD41. Analysis in a FACScan flow cytometer revealed that the corresponding populations were more than 99% glycophorin A and CD41-positive, respectively.

A mixture of granulocytes, monocytes, and lymphocytes was obtained by lysing the erythrocytes using Optilyse™ reagent (Immunotech) according to the manufacturer's instructions. The three fractions were analyzed separately by appropriate gate setting in a dual scatter plot on a FACScan flow cytometer. Prior to analysis, normal mononuclear BM cells and leukemic cells (from PB) were purified by collecting the interphase cells on a Ficoll-Hypaque gradient.

*Purification of CD34-positive Bone Marrow Cells*

CD34-positive bone marrow cells were purified on a Ceprate™ LC CD34 affinity column (CellPro, Bothell, WA). Gravity flow separation of approximately 8 x $10^7$ mononuclear cells was performed according to the manufacturer's

instructions. Approximately $9 \times 10^5$ cells containing 81% of CD34-positive cells were collected after separation. Positivity was estimated using the CD34-reactive antibody HPCA-2-FITC (Becton Dickinson, Heidelberg).

*Immunofluorescence Staining*

Immunofluorescence staining was carried out using (phycoerythrin) PE conjugated sheep anti-mouse serum. Unspecific binding to Fc receptors was blocked by incubating cells with 10% human AB serum (Behring, Marburg, Germany) for 10 minute on ice. In the next step, cells were incubated with the designated antibody (2 μg/ml) for 15 minutes, washed, and stained using the $F(ab')_2$ fragment of a PE conjugated sheep anti-mouse serum (Sigma, München, Germany). Background staining was performed with an IgG1 control antibody (Southern Biotechnology). CD34-positive cells were labeled with mAb 24D2 (IgG1) and stained with an isotype-matched goat anti-mouse antibody conjugated with PE (Southern Biotechnology).

*Flow Cytometric Analysis*

Flow cytometric analysis was performed on a FACScan flow cytometer (Becton Dickinson) equipped with an argon ion laser. Cell fluorescence was excited at 488 nm and the phycoerythrin (PE) signals were detected through a 570 nm band pass filter. Ten-thousand events were acquired and analyzed on an Amatic 486 PC.

Results

*HER-2 Protein Expression on Leukemia blasts*

Blasts from infant and adult patients suffering from chronic or acute leukemia were analyzed on a flow cytometer for their cell surface expression of HER-2 protein. As shown in Table 1 the cells from 2/15 infant and 9/19 adult patients with C-ALL expressed HER-2 protein on their surface. One out of two ALL (CD19+ CD10-), and 3/4 CML-BC samples with a C-ALL phenotype (CD19+ CD10+), expressed HER-2 protein. No expression of HER-2 protein was observed on the blasts from patients with T-ALL (0/3 samples from infant patients and 0/7 samples from adult patients), AML (0/30), CLL (0/5), CML in chronic or accelerated phase (0/5) or in blast crisis (CML-BC) with myeloid differentiation (0/30).

TABLE 1

| Expression of HER-2 Protein on Leukemia Blasts | | |
|---|---|---|
| Leukemia Blast | HER-2 expression / Number tested | Description |
| AML | 0/30 | |
| T-ALL | 0/10 | 3 Infant Patients<br>7 Adult Patients |
| C-ALL | 2/15 | Infant |
| C-ALL | 9/19 | Adult |
| Pro-B ALL | 1/2 | CD19+ CD10- |
| CML | 0/5 | Chronic and accelerated phase |
| CML-BC-M | 0/30 | myeloid blast crisis |
| CML-BC-B | 3/4 | CD10+ B-lymphoblastic blast crisis |
| CLL | 0/5 | CD5+ |

At least 80% of the tested samples were made up of leukemia blasts. Monoclonal antibody 24D2 was used for detecting HER-2 protein expression. Cells were labeled with mAb 24D2, stained with a PE-conjugated anti-mouse antiserum, and analyzed on a FACScan flow cytometer.

*HER-2 Protein Expression on Normal Hematopoietic Cells*

No expression of HER-2 protein was detected on peripheral blood lymphocytes, monocytes, granulocytes, erythrocytes and platelets, on normal bone marrow cells, and on CD34-enriched normal bone marrow cells.

## Example 2: Down-Regulation of HER-2 protein on Leukemia Blasts

Down-regulation of HER-2 protein activity was studied using cells of a C-ALL sample obtained from a patient in Example 1. The chosen C-ALL sample showed the highest level of HER-2 protein expression of those blasts tested in Example 1. Antibodies were obtained as described in Example 1 and antibody-mediated down-regulation was estimated using a slight modification of the protocol described by Bühring *et al., Cancer Res. 53*:4424, 1993.

Briefly, cells were incubated in PBS for two hours at 37°C with either a control antibody or 5 $\mu$g/ml of HER-2-reactive mAB 13A1 or 13D1. After blocking and washing with ice-cold PBS, containing 0.1% $NaN_3$, cells were labeled with either an IgG1 control antibody or with 13A1 or 13D1, respectively, and stained for fluorescence analysis using a flow cytometer as described in Example 1.

Blasts treated only with the control antibody and stained with mAB 13A1 or 13D1 showed the same level of HER-2 expression as untreated cells. In contrast, about 90% of the blasts pre-incubated with mAB 13A1 and 13D1 lost HER-2 protein expression on their surface, while 10% of the cells remained positive for HER-2 protein expression. A prerequisite of a functioning HER-2 receptor is its potential to be down-regulated. Thus, the data indicates that HER-2 protein found on leukemic cells is a functioning HER-2 receptor.

## Example 3: HER-2 Protein Expression in Hematopoietic Cells Lines

Expression of HER-2 protein on normal and leukemic cell lines was measured using antibodies targeted to HER-2 protein as described in Example 1. Hematopoietic cell lines were obtained from different sources. K562 and HEL (erythroleukemias), KG-1, KG-1a, HL-60, and U937 (myeloid leukemias), HSB-2, CCRF-CEM, and Molt-4 (T-lymphoblastic leukemias), Daudi, Reh, and Cess (B-lymphoblastoid leukemias) were obtained from the American Type Culture Collection (ATCC). The cell lines M07-e (megakaryoblastic leukemia), TMM, Nalm-1, BV-173, CML-T1, SPI-801, and EM-2 (derived from CML), and U-266 (myeloma) were purchased from "The German Collection of Microorganisms and Cell Cultures" (DSM). Additionally the following cells lines were obtained from different sources: were UT-7 (erythroleukemia) (*see*, Komatsu *et al., Canc. Res. 51*:341, 1991), MEG-01 (megakaryoblastic) (*see*, Ogura *et al., Blood 66*:1384, 1985) TF-1 (erythroleukemic) (*see*, Kitamura *et al., Cell Physiol. 140*:323, 1989), MOLM-1 (megakaryoblastic) (*see*, Matsuo *et al., Human Cell 4*:261, 1991), DU.528 (myeloid) (*see*, Begley *et al., Proc. Natl. Acad. Sci. USA 86*:2031, 1989), OCI/AML-4 (myeloid) (*see*, Koistinen *et al., Leukemia 5*:704, 1991, and Km3 (pre-B-lymphoblastoid).

The results of testing leukemic cell lines of various lineages for expression of HER-2 protein is shown in Table 2.

TABLE 2

| EXPRESSION OF HER-2 PROTEIN ON DIFFERENT HEMATOPOIETIC CELL LINES | | |
|---|---|---|
| Cell Line | HER-2 | Characteristics of Cell Line |
| *Erythroblastic/megakaryoblastic cell lines* | | |
| UT-7 | - | erythroblastic/megakaryoblastic leukemia |
| TF-1 | - | erythroleukemic leukemia |
| MO7-e | - | megakaryoblastic leukemia |
| MEG-01 | - | megakaryoblastic leukemia Ph+(CML) |
| HEL | - | erythroblastic/megakaryoblastic leukemia |
| K562 | - | erythroblastic leukemia Ph+(CML) |
| SPI-801 | - | erythroblastic leukemia (K562 derived) Ph+(CML) |
| MOLM | - | megakaryoblastic leukemia Ph+(CML) |
| *Myeloid cell lines* | | |
| EM-2 | - | myeloblastic leukemia Ph+(CML) |
| KG-1 | - | myeloblastic leukemia |
| KG-1a | - | immature subline of KG-1 |
| HL-60 | - | myeloblastic leukemia |
| DU.528 | - | myeloblastic leukemia (mixed phenotype) |
| U937 | - | myeloblastic leukemia |
| OCI/AML-4 | - | myeloblastic leukemia |
| *B-Cell lines* | | |
| TMM | - | B-lymphoblastic leukemia (CD10-) Ph-(CML) |
| BV-173 | + | B-lymphoblastic (CD10+) Ph+(CML) |
| Nalm-1 | + | B-lymphoblastic leukemia (CD10+) Ph+(CML) |
| Daudi | - | B-lymphoblastic leukemia |
| Reh | - | pre B-lymphoblastic leukemia (CD10+) |
| U266 | - | myeloma |
| Km3 | - | pre B-lymphoblastic leukemia (CD10+) |
| Cess | - | C-lymphoblastic leukemia |
| *T-cell lines* | | |
| CML-T1 | - | T-lymphoblastic leukemia Ph+(CML) |
| HSB-2 | - | T-lymphoblastic leukemia |
| CCRF-CEM | - | T-lymphoblastic leukemia |
| Molt-4 | - | T-lymphoblastic leukemia |

Cells were labeled with mAb 24D2, stained with a PE-conjugated anti-mouse antiserum, and analyzed on a FACScan flow cytometer. Only the B-lymphoblastic cell lines BV-173 and Nalm-1 had detectable levels of HER-2 protein surface expression. Between BV-173 and Nalm-1, BV-173 had a higher expression level. Additionally, BV-173 had cell surface expression of EGF-R while Nalm-1 did not express EGF-R.

Other embodiments are within the following claims.

## Claims

1. An ex vivo-method of inhibiting proliferation of a leukemic cell derived from a patient suffering from acute lymphatic leukemia (ALL) comprising the step of exposing said cell to a proliferation inhibiting amount of an anti-HER-2 agent, wherein said cell expresses HER-2 protein.

2. The method of claim 1, wherein said leukemic cell is of B-lymphoblastic lineage.

3. The method of claim 1, wherein said cell expresses CD19.

4. The method of claim 1, wherein said cell expresses CD10.

5. The method of claim 1, wherein said cell expresses CD10 and CD19.

6. The method of claim 1, wherein said cell expresses CD19 but not CD10.

7. The method of claim 1 or 2, wherein said anti-HER-2 agent is selected from the group consisting of: HER-2 inhibiting compound, anti-HER-2 nucleic acid, anti-HER-2 antibody, and defective HER-2 protein.

8. An ex vivo-method of treating hematopoietic cells obtained from a leukemia patient suffering from acute lymphatic leukemia (ALL), said cells containing leukemic cells expressing HER-2 protein, comprising the step of filtering out said leukemic cells from normal hematopoietic cells.

9. The method of claim 8, wherein said method filters said leukemic cells using an antibody which binds HER-2 protein.

10. An ex vivo-method for determining whether a patient has leukemic cells originating from acute lymphatic leukemia (ALL), characterized by cell surface expression of HER-2 protein, comprising the steps of:

    a) providing a HER-2 binding agent to hemapoietic cells containing said leukemic cells from said patient; and
    b) measuring binding of said agent to said leukemic cells.

11. The method of claim 10, wherein said binding agent is an antibody which binds HER-2.

12. An ex vivo-method for determining whether proliferation of a leukemic cell in a patient suffering from acute lymphatic leukemia (ALL) is due to HER-2 protein activity, comprising the steps of:

    a) exposing said cell removed from said patient to a cell proliferation inhibiting amount of an anti-HER-2 agent, and
    b) measuring the growth of said cell after exposure to said agent.

13. The method of claim 12, wherein said cell expresses a polypeptide selected from the group consisting of: CD10 and CD19.

## Patentansprüche

1. Ex vivo-Verfahren zur Inhibition der Proliferation einer Leukämiezelle, die aus einem an akuter lymphatischer Leukämie (ALL) leidenden Patienten stammt, mit dem Schritt, die Zelle einer die Proliferation inhibierenden Menge eines Anti-HER-2-Agens auszusetzen, wobei die Zelle HER-2 Protein exprimiert.

2. Verfahren nach Anspruch 1, bei dem die Leukämiezelle der B-lymphoblastischen Linie angehört.

3. Verfahren nach Anspruch 1, bei dem die Zelle CD19 exprimiert.

4. Verfahren nach Anspruch 1, bei dem die Zelle CD10 exprimiert.

5. Verfahren nach Anspruch 1, bei dem die Zelle CD10 und CD19 exprimiert.

6. Verfahren nach Anspruch 1, bei dem die Zelle CD19, jedoch nicht CD10 exprimiert.

7. Verfahren nach Anspruch 1 oder 2, bei dem das Anti-HER-2-Agens ausgewählt ist aus der Gruppe bestehend aus einer HER-2 inhibierenden Verbindung, einer Anti-HER-2-Nukleinsäure, einem Anti-HER-2-Antikörper und einem defekten HER-2-Protein.

8. Ex vivo-Verfahren zur Behandlung hämatopoetischer Zellen, die aus einem an akuter lymphatischer Leukämie (ALL) leidenden Patienten gewonnen wurden, wobei die Zellen Leukämiezellen umfassen, die HER-2-Protein exprimieren, mit dem Schritt, die Leukämiezellen aus normalen hämatopoetischen Zellen herauszufiltern.

9. Verfahren nach Anspruch 8, bei dem das Verfahren die Leukämiezellen unter Verwendung eines HER-2-Protein bindenden Antikörpers herausfiltert.

10. Ex vivo-Verfahren zur Bestimmung, ob ein Patient Leukämiezellen hat, die aus einer akuten lymphatischen Leukämie (ALL) stammen, gekennzeichnet durch eine Zelloberflächenexpression des HER-2-Proteins, mit den Schritten:

   a) Zugabe eines HER-2-bindenden Agens zu hämatopoetischen Zellen, die die Leukämiezellen aus dem Patienten umfassen; und

   b) Messung der Bindung des Agens an die Leukämiezellen.

11. Verfahren nach Anspruch 10, bei dem das bindende Agens ein Antikörper ist, der HER-2 bindet.

12. Ex vivo-Verfahren zur Bestimmung, ob die Proliferation einer Leukämiezelle eines an akuter lymphatischer Leukämie (ALL) leidenden Patienten auf eine HER-2-Protein-Aktivität zurückzuführen ist, mit den Schritten:

   a) die aus dem Patienten entnommene Zelle wird einer die Zellproliferation inhibierenden Menge eines Anti-HER-2 Agens ausgesetzt; und

   b) das Wachstum der Zelle wird gemessen, nachdem die Zelle dem Agens ausgesetzt wurde.

13. Verfahren nach Anspruch 12, bei dem die Zelle ein Polypeptid exprimiert, das ausgewählt ist aus der Gruppe bestehend aus CD10 und CD19.

**Revendications**

1. Procédé *ex vivo* pour inhiber la prolifération d'une cellule leucémique issue d'un patient atteint de leucémie aiguë lymphoblastique (LAL), comprenant l'étape consistant à exposer ladite cellule à une quantité inhibant la prolifération d'un agent anti-HER-2, dans lequel ladite cellule exprime la protéine HER-2.

2. Procédé selon la revendication 1, dans lequel ladite cellule leucémique appartient à la lignée B-lymphoblastique.

3. Procédé selon la revendication 1, dans lequel ladite cellule exprime CD19.

4. Procédé selon la revendication 1, dans lequel ladite cellule exprime CD10.

5. Procédé selon la revendication 1, dans lequel ladite cellule exprime CD10 et CD19.

6. Procédé selon la revendication 1, dans lequel ladite cellule exprime CD19 mais pas CD10.

7. Procédé selon la revendication 1 ou 2, dans lequel ledit agent anti-HER-2 est choisi dans le groupe constitué d'un composé inhibant HER-2, d'un acide nucléique anti-HER-2, d'un anticorps anti-HER-2, et d'une protéine HER-2 défectueuse.

8. Procédé *ex vivo* pour traiter des cellules hématopoïétiques obtenues chez un patient leucémique atteint de leucémie aiguë lymphoblastique (LAL), lesdites cellules contenant des cellules leucémiques exprimant la protéine HER-2, comprenant l'étape consistant à séparer par filtration lesdites cellules leucémiques des cellules hématopoïétiques normales.

**9.** Procédé selon la revendication 8, dans lequel ledit procédé filtre lesdites cellules leucémiques au moyen d'un anticorps qui se lie la protéine HER-2.

**10.** Procédé *ex vivo* pour déterminer si un patient possède des cellules leucémiques provenant d'une leucémie aiguë lymphoblastique (LAL), caractérisée par l'expression à la surface des cellules de la protéine HER-2, comprenant les étapes consistant à :

   a) fournir un agent de liaison à HER-2, à des cellules hématopoïétiques contenant lesdites cellules leucémiques dudit patient; et
   b) mesurer la liaison dudit agent auxdites cellules leucémiques.

**11.** Procédé selon la revendication 10, dans lequel ledit agent de liaison est un anticorps qui se lie à HER-2.

**12.** Procédé *ex vivo* pour déterminer si la prolifération d'une cellule leucémique chez un patient atteint de leucémie aiguë lymphoblastique est causée par l'activité de la protéine HER-2, comprenant les étapes consistant à :

   a) exposer ladite cellule retirée dudit patient à une quantité inhibant la prolifération cellulaire d'un agent anti-HER-2, et
   b) mesurer la croissance de ladite cellule après exposition audit agent.

**13.** Procédé selon la revendication 12, dans lequel ladite cellule exprime un polypeptide choisi dans le groupe constitué de CD10 et de CD19.